# EUROPEAN PATENT APPLICATION

(11) **EP 2 418 208 A1**
(43) Date of publication of application: **15.02.2012**
(21) Application number: 10761463.8
(22) Date of filing: 09.04.2010
(51) Int. Cl.: C07D 311/62, A23L 1/30, A61K 31/353, A61K 36/18, A61P 1/04, A61P 1/14, A61P 43/00

(54) **INHIBITOR FOR ELEVATION OF GIP LEVEL**

(30) Priority: 10.04.2009 JP 2009096063
(71) Applicant: Kao Corporation, Chuo-Ku Tokyo 103-8210 (JP)
(72) Inventor: ENOKUCHI, Yuka, Haga-gun Tochigi 321-3497 (JP); MURASE, Takatoshi, Haga-gun Tochigi 321-3497 (JP)
(74) Representative: HOFFMANN EITLE
(86) International application number: PCT/JP2010/002604
(87) International publication number: WO 2010/116760

(57) **Abstract**

Provided is a GIP-increase inhibitor, which is customary used for food production, is excellent in safety, and is useful for, for example, a medicine or a food. The GIP-increase inhibitor contains catechins as active ingredient.

## Description

### Field of the Invention

The present invention relates to a GIP-increase inhibitor, which is useful for a medicine or a food.

### Background of the Invention

Gastric inhibitory polypeptide/glucose-dependent insulinotropic peptide (GIP) is a gastrointestinal hormone formed of 42 amino acids, which was discovered in 1973. It is known that GIP is secreted from a K cell distributed in the stomach, the small intestine and the upper part of the duodenum after ingestion of nutrients such as carbohydrate or lipid into a body, and shows a gastric acid secretion inhibitory action and a gastric motility inhibitory action (Non Patent Documents 1 to 3).

Further, because a GIP receptor is expressed in the small intestine, heart, vascular endothelial cell, bone, spleen, lung, kidney, thyroid, central nervous system, and the like, controlling the amount of GIP is expected to enable the adjustment of various functions via GIP in tissues throughout the entire body, such as a gastrointestinal function, a cardiac function, a vascular function, a bone function, a lung function, a kidney function, a thyroid function, and a central nervous function, accordingly, a number of studies for GIP secretion and control of a GIP function have been made, and relevant materials have been widely searched.

According to previous studies, 3-bromo-5-methyl-2-phenylpyrazolo[1,5-a]pyrimidin-7-ol (BMPP) is found as a substance inhibiting the functions of GIP, and guar gum and the like are reported to be substances which inhibit the secretion of GIP (Patent Document 1 and Non Patent Documents 4 to 9). In addition, in recent years, as food ingredients showing a GIP-decrease action, a diacylglycerol (Patent Document 2), alginic acid or a salt thereof, or hydroxypropyl starch (Patent Document 3), a monoacylglycerol (Patent Document 4), and the like, have been reported.

Meanwhile, catechins are contained in tea, cocoa, or the like, and are food ingredients which have been widely ingested all over the world for a long time, and are excellent in safety. Functions of the catechins, including, for example, an antioxidative action (Non Patent Document 10), an antitumor action (Non Patent Document 11), an amylase inhibitory action (Patent Document 5), a hypolipidemic action (Patent Document 6 and Non Patent Document 12), a blood pressure regulating action (Patent Document 7), an endurance improving action (Patent Document 8 and Non Patent Document 13), ananti-fatigueaction (Patent Document 9), an anti-aging action (Patent Document 10), a muscle hypofunction inhibitory action (Patent Document 11), an ameliorating action on an allergic symptom (Patent Documents 12 and 13), a gastric mucosa protecting action (Non Patent Document 14), and a digestive enzyme activating action (Non Patent Document 15) have been reported. However, relationship between the catechins and the GIP secretion has not been so far reported.

### Prior Arts Documents

### Patent Documents

[Patent Document 1] JP-A-2002-37744
[Patent Document 2] JP-A-2006-342084
[Patent Document 3] JP-A-2006-342085
[Patent Document 4] JP-A-2007-290989
[Patent Document 5] JP-A-2008-138129
[Patent Document 6] JP-A-2006-1909
[Patent Document 7] JP-A-2007-70338
[Patent Document 8] JP-A-2005-89384
[Patent Document 9] JP-A-2007-191426
[Patent Document 10] JP-A-2008-63318
[Patent Document 11] JP-A-2008-13473
[Patent Document 12] JP-A-2007-31314
[Patent Document 13] JP-A-2007-186462

### Non Patent Documents

[Non-Patent Document 1] Can J. Physiol. Pharmacol., (1969), 47 p. 113-114
[Non-Patent Document 2] J. Clin. Endocrinol. Metab., (1973), 37, p. 826-828
[Non-Patent Document 3] Chugai-Igakusha, Digestive Tract-Functions and Pathological conditions ("Shoukakan Kinou to Byoutai"), (1981), p. 205-216
[Non-Patent Document 4] Diabet. Med., (1996), 13, p. 358-364
[Non-Patent Document 5] Br. J. Nutr., (1995), 74, p. 539-556
[Non-Patent Document 6] Reprod. Nutr. Dev., (1992), 32, p. 11-20
[Non-Patent Document 7] Br. J. Nutr., (1990), 64, p 103-110
[Non-Patent Document 8] Diabet. Med., (1990), 7, p. 515-520
[Non-Patent Document 9] Br. J. Nutr., (1985), 53, p. 467-475
[Non-Patent Document 10] J. Agric Food Chem., (1996), 44, p. 3426-3431
[Non-Patent Document 11] J. Nutr., (2003), 133, p. 3268-3274
[Non-Patent Document 12] Biosci. Biotechnol. Iochem., (2007), 71, p. 971-976
[Non-Patent Document 13] Am. J. Physiol. Regul. Integr. Comp. Physiol., (2005), 288, p. R708-715
[Non-Patent Document 14] J. Agric Food Chem., (2008), 56, p. 12122-12126
[Non-Patent Document 15] J. Agric Food Chem., (2005), 53, p. 8706-7813

### Summary of the Invention

The present invention relates to provision of the following items (1) to (7).
(1) A GIP-increase inhibitor, containing catechins as active ingredient.
(2) A food or beverage for inhibiting GIP-increase, including catechins in an effective amount.
(3) A method for inhibiting GIP-increase, including administration, to a subject in need thereof, or ingestion of catechins.
(4) A catechins for use in the inhibition of GIP-increase.
(5) Use of catechins for manufacturing a GIP-increase inhibitor.
(6) The GIP-increase inhibitor according to the above-mentioned item (1), the food or beverage for inhibiting GIP-increase according to the above-mentioned item (2), the method for inhibiting GIP-increase according to the above-mentioned item (3), the catechins for the inhibition of GIP-increase according to the above-mentioned item (4), or the use of catechins for manufacturing a GIP-increase inhibitor according to the above-mentioned item (5), in which the catechins include one or more kinds of ingredients selected from the group consisting of catechin, catechin gallate, gallocatechin, gallocatechin gallate, epicatechin, epigallocatechin, epicatechin gallate, and epigallocatechin gallate.
(7) The GIP-increase inhibitor according to the above-mentioned item (1), the food or beverage for inhibiting GIP-increase according to the above-mentioned item (2), the method for inhibiting GIP-increase according to the above-mentioned item (3), the catechins for the inhibition of GIP-increase according to the above-mentioned item (4), or the use of catechins for manufacturing a GIP-increase inhibitor according to the above-mentioned item (5), in which the catechins include tea catechins.

### Brief Description of Drawings

[FIG. 1] FIG. 1 shows a change of a blood GIP level (pg/mL) over time after administration in each of a tea catechin A group (administration of tea catechin A) and a control group (no administration of a catechin).
[FIG. 2] FIG. 2 shows a change of a blood GIP level (pg/mL) ten minutes after administration in each of a tea catechin B group (administration of tea catechin B), a tea catechin C group (administration of tea catechin C), and a control group (no administration of a catechin).

### Description of Embodiments

The present invention relates to providing a GIP-increase inhibitor, which is customary used for food production, is excellent in safety, and is useful as an active ingredient of, for example, a medicine or a food.

The inventors of the present invention have studied various substances capable of decreasing a blood GIP level, and as a result, have found that catechins show an excellent GIP-increase inhibitory action, and can be used as an active ingredient blended into a product such as, for example, a pharmaceutical agent and food and beverage, which shows the action.

A GIP- increase inhibitor or catechins of the present invention shows, for example, an excellent postprandial GIP-increase inhibitory action, an excellent postprandial gastric acid secretion improving action and an excellent postprandial heavy feeling improving action in the stomach. Therefore, the GIP-increase inhibitor or catechins according to the present invention is useful for inhibiting GIP-increase; for lowering, preventing, or improving the risk of developing a disease whose symptoms are aggravated by GIP-increase; and is useful as an active ingredient contained in, for example, a pharmaceutical agent, food and beverage, or pet food which is used for, for example, improvement of a gastric acid secretion ability or a heavy feeling in the stomach. Further, controlling the amount of GIP is expected to enable the adjustment of various functions via GIP in tissues throughout the entire body, such as a gastrointestinal function, a cardiac function, a vascular function, a bone function, a lung function, a kidney function, a thyroid function, and a central nervous function. The GIP-increase inhibitor or catechins is thus useful as a material for the adjustment.

The catechins according to the present invention contains one or more kinds selected from the group consisting of non-epi-form catechins such as catechin, catechin gallate, gallocatechin, and gallocatechin gallate, and epi-form catechins such as epicatechin, epigallocatechin, epicatechin gallate, and epigallocatechin gallate.
The content of gallate-form catechins in the catechins is, for improving a GIP-increase inhibitory effect, preferably 10 to 100 mass% in the catechins, more preferably 20 to 100 mass%, even more preferably 25 to 100 mass%.

The catechins used in the present invention can be obtained by direct extraction from a plant material such as tea leaves and cacao beans, or by enrichment or purification of the extract, or catechins derived from any other material, a commercially available catechins, a column-purified catechins, or a chemically synthesized catechins, may be used.

Extraction of catechins from tea leaves can be carried out by adding cold water or hot water (from 0 to 100°C) to processed tea leaves derived from fresh tea leaves belonging to the genus *Camellia* such as *C*. *sinensis* or *C*. *assamica,* or a hybrid thereof, additionally by adding, if needed, an extraction aid to the mixture, and extracting the catechins. Further, an extraction method under a so-called non-oxidative atmosphere, created by eliminating dissolved oxygen by boiling deaeration or aeration of inert gas such as a nitrogen gas, may be used in combination.
The processed tea leaves include, for example, (1) green tea leaves such as sencha, bancha, gyokuro, tencha, and kamairicha, (2) half fermented tea leaves such as tieguanyin, shikishu, golden osmanthus, and buigancha, which are collectively called oolong tea, and (3) fermented tea leaves such as Darjeeling, Uva, and Keemun tea, which are called black tea.
Examples of the extraction aid include an organic acid such as sodium ascorbate or organic acid salts thereof.

Further, commercially available product may be used as the catechins of the present invention, in addition to the above-mentioned catechins extracted/purified from tea leaves. For example, a catechin preparation such as Polyphenon (Mitsui Norin Co., Ltd.), Thea-flan (Ito En, Limited), Sunphenon (Taiyo Kagaku Co. , Ltd.), and Teavigo (DMS Nutritional Products Ltd.) may be used.
The enrichment of the tea extract can be carried out by concentrating the above-mentioned extract. The purification of the tea extract can be carried out by purifying the above-mentioned extract by using a solvent or a column. Examples of form of the concentrated or purified tea extract include various forms such as a solid, an aqueous solution, and a slurry.
For example, the tea extract can be prepared by methods exemplified, for example, in JP-A-1984-219384, JP-A-1992-20589, JP-A-1993-260907, and JP-A-1993-306279 in detail.

Further, catechins or the like derived from a plant extract has peculiar bitterness and astringency, and hence, when the present invention is used in a food or the like, the taste should preferably be improved in some cases. In particular, a tea extract contains, in addition to a catechin, a number of ingredients such as an organic acid, a protein, and plant fiber, which produce a bitter taste or harshness peculiar to tea, and hence the taste can be improved by enhancing the purity of the catechins. When a plant extract, in particular, a green tea extract is used, it is desired that the purity of the catechins is 20 mass% or more of the extract, and the purity of the catechins is more preferably 30 mass% or more, even more preferably 30 to 95 mass%.
Further, the content of gallate-form catechins is, for theimprovement of taste of a food or the like, is preferably 10 to 100 mass% of the catechins, more preferably 20 to 90 mass%, more desirably 25 to 80 mass%. The content ratio of the gallate-form catechins in the catechins can be adjusted by a method such as tannase treatment (JP-A-2007-61083).

Further, when a plant extract, in particular a tea extract, is used as the catechins, the extract contains caffeine. Caffeine shows awakening action, therefore the caffeine shows the effects of preventing and improving, for example, sleepiness, malaise, and headache. However, some persons exhibit a hypersensitivity reaction to caffeine and some other persons do not like caffeine, therefore, the content of caffeine in the plant extract maybe reduced.
Accordingly, depending on the use purpose of the preparation of the present invention, it is possible to use the sole catechin or a plant extract in combination with caffeine, or a plant extract having lower content of caffeine by performing decaffeination. Caffeine provides a bitter taste, and hence, particularly when decaffeinated catechins is used in a food or beverage, it is desired that the content of caffeine is 20 mass% or less with respect to the catechins, preferably 5 mass% or less, more preferably 2 mass% or less, even more preferably 1 mass% or less.
A method of producing tea catechins composition having low content of caffeine is described in, for example, JP-A-2006-8580, JP-A-2006-160656, JP-A-2006-206483, JP-A-2008-79609, and JP-A-2008-141987.

The phrase "GIP-increase inhibition" according to the present invention refers to the inhibition of the increase of the blood level of GIP secreted from the gastrointestinal tract, after ingestion of a meal (diet) containing nutrients such as lipids and carbohydrates (glucides) including glucides, particularly ingestion of a meal (diet) containing a large amount of lipids, and more particularly ingestion of a meal (diet) containing a large amount of triacylglycerols. That is, the phrase "GIP-increase inhibition" refers to the inhibition of GIP-increase mainly occurring after eating. Further, the phrase "GIP-increase inhibitory action" according to the present invention refers to a concept that includes both a GIP secretion inhibitory action in which GIP-increase is inhibited by the inhibition of GIP secretion from the gastrointestinal tract, and a GIP-decrease action in which GIP-increase is inhibited by decreasing a blood GIP concentration.

The catechins of the present invention have an excellent blood GIP-increase inhibitory action as shown in the examples described below. Further, as described above (see Non Patent Documents 1 to 3), inhibition of GIP-increase alleviates the inhibition of gastric acid secretion and the inhibition of gastric motility. Thus, the catechins can be used, by feeding or administering it to animals including humans, for a method of inhibiting blood GIP-increase, methods of lowering, preventing, and improving the risk of developing a disease whose symptoms are aggravated by GIP-increase, and methods of improving a gastric acid secretion ability and improving a heavy feeling in the stomach. Further, the catechins can also be used for a GIP-increase inhibitor, agents for lowering, preventing, and improving the risk of developing a disease whose symptoms are aggravated by GIP-increase, and agents for improving a gastric acid secretion ability and improving a heavy feeling in the stomach (hereinafter referred to as "GIP-increase inhibitor or the like"), and hence the catechins can be used as an active ingredient for those preparations. Further, the catechins can be used for manufacturing the GIP-increase inhibitor, the agents for lowering, preventing, and improving the risk of developing a disease whose symptoms are aggravated by GIP-increase, and the agents for improving a gastric acid secretion ability and improving a heavy feeling in the stomach.
In this case, the GIP-increase inhibitor or the like may be manufactured by using the catechins alone, or by combination with, in addition to the catechins, any of the below-mentioned preparations acceptable for blending, such as a carrier, the acceptable preparations being chosen arbitrarily if necessary. These preparations can be manufactured by using an ordinary method according to the purpose of use.

Besides, the GIP-increase inhibitor or the like can be used as an active ingredient in, for example, various kinds of foods and beverages, pharmaceutical agents, quasi drugs, and pet food which are produced for inhibiting GIP-increase, for improving a heavy feeling in the stomach of humans or non-human animals.
Further, the GIP-increase inhibitor or the like can be used as an active ingredient in functional food such as health food, beauty food, medical food, food for specified health use, and nutraceutical and functional food , wherein the functional food express the concept of containing catechins and showing a GIP secretion inhibitory action or the like, and , if necessary, a sign indicating that it is used for, for example, inhibiting GIP-increase, lowering, preventing, and improving the risk of developing a disease whose symptoms are aggravated by GIP-increase, improving a gastric acid secretion ability and improving a heavy feeling in the stomach.

When the GIP-increase inhibitor or the like of the present invention is used as an active ingredient in a pharmaceutical agent or a quasi drug, the pharmaceutical agent or the quasi drug may be administered in any administration form.
Examples of the administration form include oral administration, enteral administration, transmucosal administration, and administration with an injection. Examples of the dosage form for oral administration include oral solid formulations such as a tablet, a coated tablet, a capsule, a granule, an abstract, a powder, a sustained-release preparation, a sugar coatedtablet, apill, and a fine granule; and oral liquid formulations such as a suspension, an emulsion, an internal use liquid, an internal use liquid preparation, a syrup, and an elixir.
Examples of the dosage form for parenteral administration include an intravenous injection, an intramuscular injection, an inhalant, an infusion, a suppository, an inhalant, an epicutaneous drug, an eye drop, and a nasal drop.

In such formulations, the catechins may be used alone or in combination with a pharmaceutically acceptable carrier. Examples of such carrier include an excipient, a binder, a disintegrant, a lubricant, a diluent, an osmotic pressure adjuster, a fluidity promoter, an absorption aid, a pH adjuster, an emulsifier, an antiseptic, a stabilizer, an antioxidant, a colorant, a UV absorber, a moisturizer, a thickner, a brightening agent, an activity enhancer, an anti-inflammatory agent, a germicide, a taste masking agent, an odor masking agent, a filler, a surfactant, a dispersant, a buffer, a preservative, a flavoring, and a coating agent.

In the case of preparing an oral solid formulation, the preparation of the present invention may be mixed with an excipient and, for example, as required, a binder, a disintegrant, a lubricant, a colorant, a taste masking agent or an odor masking agent, and then treated by a conventional method, to thereby produce a tablet, a coated tablet, a granule, an abstract or a capsule. Further, when an oral liquid formulation is prepared, the preparation may be mixed with a taste masking agent, a buffer, a stabilizer or an odor masking agent, and then treated by a conventional method, to thereby produce an internal use liquid, a syrup or an elixir.

When the GIP-increase inhibitor or the like of the present invention is used as an active ingredient in a food or beverage, its form may be a solid, a semi-solid, or a liquid. Examples of the food or beverage include bread, noodles, confectionary such as a coolie, jellies, a dairy product, a frozen food, a convenience food, a starch processed product, a processed meat product, other processed foods, beverages such as Oolong tea, green tea, black tea, and coffee, soups, seasonings, and a dietary supplement, and raw materials thereof. Further, the form may be, as is the case of the above-mentioned oral formulation, a tablet, a pill, a capsule, a solution, a syrup, a powder, a granule, or the like.

In order to prepare those foods and beverages in various forms, the catechins may be used alone, or may be used in appropriate combination with, for example, other food materials, a solvent, a softener, oil, an emulsifier, an antiseptic, a fragrance, a stabilizer, a colorant, a UV absorber, an antioxidant, a humectant or a thickener.

Further, when the GIP-increase inhibitor or the like of the present invention is used as an active ingredient in pet food, various forms of pet food may be prepared by using raw materials similar to those used for preparing the foods and beverages described above.

The blending ratio of the catechins with respect to each of the above-mentioned foods and beverages, pharmaceutical agents, and quasi drugs varies depending on their use forms. The content of the catechins in a beverage is preferably 0.01 to 5 mass%, more preferably 0.05 to 1 mass%, even more preferably 0.1 to 0.5 mass%. Further, the content of the catechins in a food usually is preferably 0.01 to 20 mass%, more preferably 0.01 to 5 mass%, even more preferably 0.1 to 2 mass%. Further, the content of the catechins in a supplement usually is preferably 0.01 to 90 mass%, more preferably 0.1 to 70 mass%, even more preferably 1 to 50 mass%.
The content of the catechins in a pharmaceutical agent, for example, an oral solid preparation such as a tablet, a granule, or a capsule usually is preferably 0.01 to 95 mass%, more preferably 5 to 90 mass%, even more preferably 20 to 50 mass%. The content of the catechins in an oral liquid preparation such as an internal use liquid or a syrup usually is preferably 0.01 to 50 mass%, more preferably 0.1 to 10 mass%, even more preferably 0.1 to 5 mass%.

The effective dosage (intake amount) of each of the above-mentioned preparations is not limited as long as good effects are provided. Further, the dosage/intake amount can vary in accordance with the condition, body weight, or sex of the subject to be dosed or other factors. The dosage/intake amount per day in terms of the catechins is preferably 100 to 5,000 mg/60 kg body weight, more preferably 300 to 2,500 mg/60 kg body weight, even more preferably 400 to 1,000 mg/60 kg body weight. The composition of the catechins is not particularly limited, but the content of gallate-form catechins is preferably larger from the standpoint of being effective. Further, each of the above-mentioned preparations can be administered in accordance with any administration regimen, is preferably administered once to several times per day by dividing the dosage of the each preparation . Further, the each preparation may be administered before meal, during meal or after meal, and should be administered preferably before meal or during meal.
A subj ect for administration or ingestion of the GIP- increase inhibitor or the like of the present invention is not particularly limited as long as the subject is in need thereof. The subject is preferably a human whose fasting blood GIP level is 30 pg/mL or more, or a human whose basic amount of gastric secretion is 30 mL/hour or less in the gastric secretion function test.

Hereinafter, examples and blending examples are shown to exemplify the present invention specifically, however, the present invention is not limited thereto.

### Examples

### Example 1 <GIP inhibitory action of tea catechin 1>

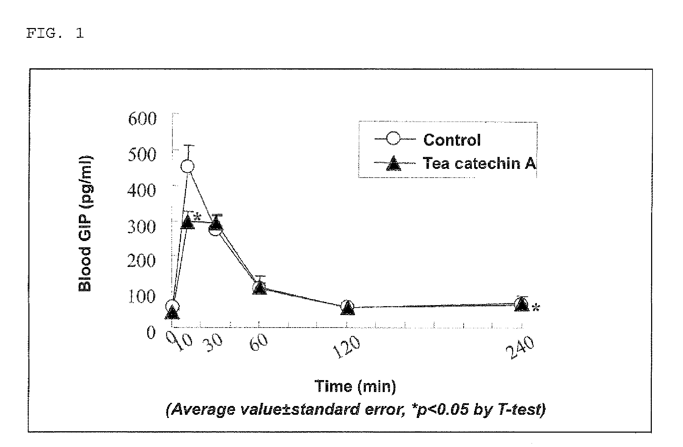

Mice (C57BL/6J male, nine weeks old) were grouped into two groups, a control group and a tea catechin A group (ten mice for each group), such that the average body weight of the mice in each group became identical. Tea catechin A was prepared by dissolving a dry powder prepared by hot water extract from green tea (*Camellia sinensis*) leaves in hot water, adding, to the resultant aqueous solution, equivalent amount of chloroform and three times amount of ethanol, followed by mixing, collecting an aqueous phase, and drying again. After fasting for 16 hours, a control solution (20 mass% ethanol) and a tea catechin A solution (1 mass%/20 mass% ethanol solution) were orally administered (100 mg/kg body weight in terms of the total amount of catechins) through a probe to the control group and the tea catechin A group, respectively. Subsequently, a starch/corn oil mixed solution (containing 2 g/kg body weight of each ingredient) emulsified with egg lecithin (Wako Pure Chemical Industries, Ltd.) (0.02 g/kg body weight) was administered to the each group.
Among all of the catechins, the composition of the tea catechin A used in this test included 3.2% of catechin, 9.7% of epicatechin, 6.9% of gallocatechin, 51.1% of epigallocatechin, 3.3% of epicatechin gallate, 1.2% of gallocatechin gallate, and 24.4% of epigallocatechin gallate (gallate-form composition 29%).
Blood was collected from a fundus of each mouse before the administration and time-dependently after the administration. The blood GIP level of the each mouse was measured by an ELISA method (Gastric Inhibitory Peptide EIA Kit, Phenix Pharmaceutical Inc.). The measurement results are shown in FIG 1.

### Example 2 <GIP inhibitory action of tea catechin 2>

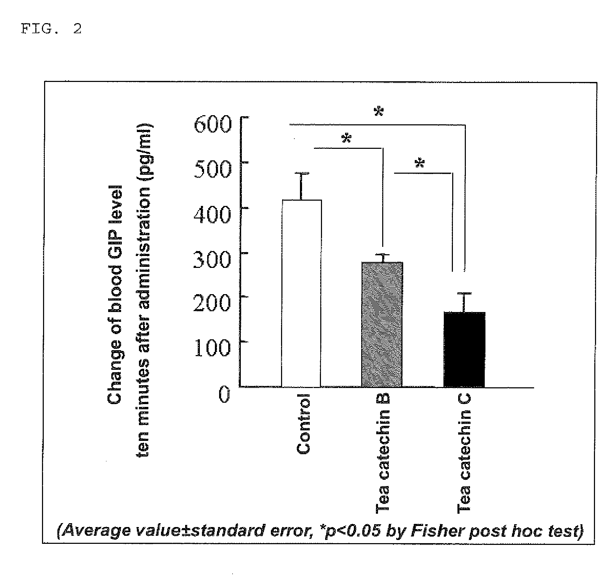

A powder prepared by subj ecting a Polyphenon HG (Mitsui Norin Co. , Ltd.) aqueous solution to heat treatment at 121°C for 20 minutes, adjusting the pH of the resultant solution to 6.5 with sodium bicarbonate, and then performing freeze-drying was used as tea catechin B. Thea-flan 90S (Ito En, Limited) was used as tea catechin C.
Mice (C57BL/6J male, nine weeks old) were grouped into three groups, a control group, a tea catechin B group, and a tea catechin C group (ten mice for each group), such that the average body weight of the mice in each group became identical. After fasting for 16 hours, a control solution (20 mass% ethanol), a tea catechin B solution (2 mass%/20 mast% ethanol solution), and a tea catechin C solution (2 mass%/20 mass% ethanol solution) were orally administered (200 mg/kg body weight in terms of the total amount of catechins) through a probe to the control group, the tea catechin B group, and the tea catechin C group, respectively. Subsequently, a starch/corn oil mixed solution (containing 4 g/kg body weight of each ingredient) emulsified with egg lecithin (Wako Pure Chemical Industries, Ltd.) (0.02 g/kg body weight) was administered to each group.
Note that the compositions of tea catechins B and C used in this test are shown in Table 1.
Blood was collected from a fundus of each mouse before the administration and 10 minutes after the administration. The blood GIP level of the each mouse was measured by an ELISA method (Gastric Inhibitory Peptide EIA Kit, Phenix Pharmaceutical Inc.). Changes of ten minutes after the administration (blood GIP level ten minutes after administration - blood GIP level before administration) are shown in FIG 2.

**[Table 1]**

| Catechin composition (%) | Tea catechin B | Tea catechin C |
|---|---|---|
| Catechin | 6 | 0 |
| Epicatechin | 7 | 0 |
| Gallocatechin | 21 | 0 |
| Epigallocatechin | 16 | 0 |
| Catechin gallate | 5 | 1 |
| Epicatechin gallate | 8 | 21 |
| Gallocatechin gallate | 16 | 5 |
| Epigallocatechin gallate | 21 | 73 |
| Composition ratio (%-) of gallate-form catechins | 50 | 100 |

As shown in PIG. 1 and FIG. 2, the administration (ingestion) of each of the tea catechins resulted in statistically significant inhibition of the increase of a blood GIP level. Thus, it can be concluded that the catechins are effective for inhibiting GIP-increase.

Blending examples of the GIP-increase inhibitor or the like of the present invention are shown below.

### Blending example 1, Chewable tablet for inhibiting GIP-increase

Based on the formulation of 45 mass% of tea catechin (98% of epigallocatechin gallate, Teavigo (DMS Nutritional Products Ltd.)), 20 mass% of lactose, 15 mass% of ascorbic acid, 10 mass% of microcrystalline cellulose, 7 mass% of citric acid, 2 mass% of magnesium stearate, and 1 mass% of aspartame, a tablet was produced in accordance with Pharmacopeia of Japan (General Rules for Preparations "Tablets"), yielding a chewable tablet containing a tea catechin.

### Blending example 2 Tablet for inhibiting GIP-increase

Based on the formulation of 40 mass% of a cocoa extract (10% of catechin), 20 mass% of lactose, 15 mass% of ascorbic acid, 10 mass% of microcrystalline cellulose, 8 mass% of cocoa powder, 4 mass% of fructose, 2 mass% of magnesium stearate, and 1 mass% of aspartame, a tablet was produced in accordance with Pharmacopeia of Japan (General Rules for Preparations "Tablets").

### Blending example 3 Capsule for inhibiting GIP-increase

There were mixed 50 mass% of tea catechin (2% of catechin, 10% of epicatechin, 7% of gallocatechin, 24% of epigallocatechin, 1% of catechin gallate, 11% of epicatechin gallate, 4% of gallocatechin gallate, and 41% of epigallocatechin gallate), 15 mass% of vitamin C, 13 mass% of cellulose, 13 mass% of lactose, 7 mass% of corn starch, and 2 mass% of tocopherol to obtain a capsule.

### Blending example 4 Granule for inhibiting GIP-increase

There were mixed 30 mass% of Sunphenon (Taiyo Kagaku Co., Ltd.), 30 mass% of fructose, 20 mass% of glucose, 10 mass% of powdered skim milk, 8 mass% of minerals (a mixture of organic or inorganic salts of Na, K, Ca, Mg, P, Cl, Fe, and the like), and 2 mass% of vitamins (a mixture of vitamins A, D, E, B1, B2, B6, B12, C, niacin, pantothenic acid, and the like). Ethanol was added in a proper amount to the mixture, followed by enough mixing, granulation, and drying, thereby yielding a granule.

### Blending example 5 Tablet for inhibiting GIP-increase

There were mixed 30 mass% of tea catechin (6% of catechin, 7% of epicatechin, 21% of gallocatechin, 16% of epigallocatechin, 5% of catechin gallate, 8% of epicatechin gallate, 16% of Gallocatechin gallate, and 21% of epigallocatechin gallate), 20 mass% of sodium malate, 15 mass% of palatinose, 15 mass% of ascorbic acid, 5 mass% of Vitamin Mix (The Nippon Koryo Yakuhin Kaisha, Ltd.), 5 mass% of microcrystalline cellulose, 5 mass% of eggshell calcium, 4 mass% of a sucrose ester, and 1 mass% of silicon dioxide, and an ordinary method was used to prepare a tablet containing catechins.

### Blending example 6 Tablet for inhibiting GIP-increase

There were mixed 25 mass% of tea catechin (3% of catechin, 10% of epicatechin, 7% of gallocatechin, 51% of epigallocatechin, 3% of epicatechin gallate, 1% of gallocatechin gallate, and 25% of epigallocatechin gallate), 25 mass% of corn starch, 25 mass% of cellulose, 15 mass% of lactose, and 10 mass% of vitamin C, and an ordinary method was used to prepare a tablet.

## Claims

1. A GIP-increase inhibitor, comprising catechins as active ingredient.

2. The GIP-increase inhibitor according to claim 1, wherein the catechins comprises one or more kinds of ingredients selected from the group consisting of catechin, catechin gallate, gallocatechin, gallocatechin gallate, epicatechin, epigallocatechin, epicatechin gallate, and epigallocatechin gallate.

3. The GIP-increase inhibitor according to claim 1, wherein the catechins are tea catechins.

4. A method for inhibiting GIP-increase, comprising administration, to a subject in need thereof, or ingestion of catechins.

5. The method of inhibiting GIP-increase according to claim 4, wherein the catechins comprise one or more kinds of ingredients selected from the group consisting of catechin, catechin gallate, gallocatechin, gallocatechin gallate, epicatechin, epigallocatechin, epicatechin gallate, and epigallocatechin gallate.

6. The method of inhibiting GIP-increase according to claim 4, wherein the catechins are tea catechins.

7. A catechins for use in the inhibition of GIP-increase.

8. The catechins for the inhibition of GIP-increase according to claim 7, wherein the catechins comprises one or more kinds of ingredients selected from the group consisting of catechin, catechin gallate, gallocatechin, gallocatechin gallate, epicatechin, epigallocatechin, epicatechin gallate, and epigallocatechin gallate.

9. The catechins for the inhibition of GIP-increase according to claim 7, wherein the catechins are tea catechins.

10. Use of catechins for manufacturing a GIP-increase inhibitor.

11. The use of catechins for manufacturing a GIP-increase inhibitor according to claim 10, wherein the catechins comprise one or more kinds of ingredients selected from the group consisting of catechin, catechin gallate, gallocatechin, gallocatechin gallate, epicatechin, epigallocatechin, epicatechin gallate, and epigallocatechin gallate.

12. The use of catechins for manufacturing a GIP-increase inhibitor according to claim 10, wherein the catechins are tea catechins.
